# EUROPEAN PATENT SPECIFICATION

(11) **EP 0 561 264 B1**
(45) Date of publication and mention of the grant of the patent: **14.08.1996**
(21) Application number: 93103810.3
(22) Date of filing: 10.03.1993
(51) Int. Cl.: C07C 69/54, C07C 67/22, C07C 67/58, C07C 67/62, C07C 67/20

(54) **Improved process for the production of methylmethacrylate monomer from acetoncyanhydrin**
Verfahren zum Herstellen von Methylmethacrylatmonomer aus Acetoncyanhydrin
Procédé de production du méthacrylate de méthyle monomère à partir de la cyanhydrine d'acétone

(30) Priority: 16.03.1992 IT MI920602
(43) Date of publication of application: 22.09.1993
(73) Proprietor: ELF ATOCHEM ITALIA S.r.l., I-20159 Milano (IT)
(72) Inventor: Giudici, Dario, I-20017 Rho (Milan) (IT); Fabris, Alessandro, I-20017 Rho (Milan) (IT)
(74) Representative: Simeoni, Lucio, Dr.

(56) References cited:
- DATABASE WPI Week 8244, Derwent Publications Ltd., London, GB; AN 82-94153 & JP-A-57 156 438
- DATABASE WPI Week 8948, Derwent Publications Ltd., London, GB; AN 89-354414 & RO-A-96 975

## Description

The present invention relates to an improved process for preparing methylmethacrylate (MMA) through the known process of acetonecyanhydrin.

The acetoncyanhydrin process for obtaining MMA occurs in two steps: in the first one methacrylamide sulphate is obtained by reacting acetoncyanhydrin and concentrated sulphuric acid; after a first stage of exothermic reaction, a transposition reaction at high temperature is carried out to obtain the methacrylamide sulphate.

The second step of the process relates to the esterification of the methacrylamide obtained in the first step in sulphuric solution, diluted with H₂O and treated with methanol in excess. See for example the Japanese patent 57156-438.

At the end of the second step MMA is separated from the reaction mixture and submitted to distillation.
More particularly the effluents from the esterification reactor are submitted to rectification to get raw methacrylate, methanol and water and a tail containing methacrylic acid which can be recycled as such or after treatment with methanol, to the esterification section. At the bottom of the reactor a solution of acid ammonium sulphate in diluted sulphuric acid is obtained which can be treated with ammonia to form the ammonium sulphate to be used as fertilizer; or burnt in a recovery plant of the sulphuric acid, which then is recycled.
The rectified methylmethacrylate is submitted to washing with water and then led into a distillation system for the purification; the methanol extracted through washing is recovered for the recycle in the esterification step.

An alternative process comprises washing, with water and ammonia, the raw monomer containing also methacrylic acid; the aqueous phase is recycled to the esterification section and the organic phase is submitted to purification. There are many processes in the art which tend to improve the quality of the separated MMA and at the same time to increase the yield thereof.
In the distillate the most important products are MMA, methanol, water, methacrylic acid (MAA) and other volatiles, high-boiling or low-boiling organic products.
The MAA is formed in the esterification step by hydrolysis of a part of the methacrylamide.

Processes to eliminate MAA are known, since its presence in the raw methylmethacrylate is objectionable due to its high tendency to polymerise.

On the other hand, this product in an industrial process is to be recycled at the esterification stage in order to avoid remarkable yield losses with reference to the starting reagents. In this respect see the South African patent SU 600/36.
It is also known to recover MAA from the raw MMA through washing with alkaline solutions which has the further purpose to recover most of the methanol in excess present in the raw MMA. This weakly alkaline extracted solution is then recycled in the esterification stage. The obtained MMA has a titre of 90-96% and can be purified by distillation. In this connection see the Rumanian patent RO 96.975 reported in Chemical Abstract 113.172923W., Vol. 113, 1990.

It is also known that the extraction can be carried out with alkaline hydroxides, such as sodium or potassium hydroxide. However also this method has some drawbacks: as a matter of fact the regeneration of the sulphuric acid diluted in the respective plants, on leaving the esterification stage, leads to the formation of residual ashes yielding drawbacks, such as incrostations and corrosions of the insulating coatings of the furnaces in the combustion plants.

This operation must necessarily occur in an industrial process in order to avoid ecologic problems relating to its disposal. On the other hand, the use of alkaline substances, such as ammonia or amines, quite volatile during the combustion for the regeneration of sulphuric acid, do not represent a solution of the problem, both for the lower extracting power of these compounds and for the system tendency to polymerise during the extraction.

It is also known in the art to try to avoid the polymerization during distillation by adding inhibitors.

One can see, e.g., the European Patent applications EP 301879 and EP 371748 by which inhibitors in liquid phase are employed consisting of phenolic inhibitors and Mn or Ce soluble compounds, and inihibitors for the vapour phase consisting of nitric oxide and ammonium salts of N-phenyl-N-nitrous hydroxylamine. Other inhibitors are the phenolic ones with Ce or Mn alkanoates of monocarboxylic acids with C₅-C₉ carbon atoms.

Particular distillation systems concern the vaporisation of the liquid phase, containing the polymerisable liquid and the subsequent transformation of the vapour into superheated vapour by heating, and subsequent condensation to avoid unwished polymerisations. See, for example, EP 46.980.

The technical problem to be solved was to find a simple system not requiring the employment of particularly complex and expensive inhibitors; and moreover to avoid that the employed inhibitors after a certain period of time could become ineffective due to polymerisations in the system. This indeed occurs with all the inhibitos used till now.
Furthermore there was the need to avoid the use of particular synergic mixtures of inhibitors, with evident charge of costs and which are also, in many cases, very toxic.
It has been unexpectedly and surprisingly found that MAA polimerisation can be avoided in the extraction phases with ammonia or amines if the system realized by the Applicant is used, as indicated in the claims and hereinafter.
Object of the present invention is therefore a process for the purification of raw methylmethacrylate obtained by reaction of acetoncyanhydrin with an excess of concentrated sulphuric acid to form methacrylamide sulphate, then a transposition reaction is carried out at high temperature for obtaining methacrylamide sulphate; the esterification of the methacrylamide is then effected in sulphuric solution by dilution with water and by using methanol in excess; washing of the raw methylmethacrylate and subsequent distillation characterized by the fact that the washing is carried out with diluted ammonia, or amines, and at least an oxidizing agent capable to react with the oxidizable products of the reaction blank but not to react susbstantially with the methylmethacrylate and with methacrylic acid and acting in the presence of inhibitors.
The effect obtained with the present invention is quite unexpected and surprising if one considers that in the conventional methods to start the polymerisation of acrylic monomers, redox systems hydroperoxide-(metha)bisulphite, hydrogen peroxide-ions iron, etc. are used.
It was in fact quite unforeseeable that the employment of oxidizing agents could avoid the polymerisation when generally it is just this the effect they produce in presence of reducing agents.

The process is normally carried out at temperatures near room temperature by using ammonia in an amount higher than the stoichiometric necessary for the complete neutralization of the methacrylic acid. The excess can vary between 10% and 100% of the stoichiometric. In general it is suitable to add ammonia in an amount such as to maintain basic the pH of the system, in particular within the limits of 7,5-9,5, which secures the best conditions of the process according to the present invention.

It is suitable to add ammonia in the diluted form in the first stage of a pluristages counter-stream extraction or, if concentrated, under strong stirring in the aqueous phase coming from the second washing stage.

It is generally preferable to start from solutions with an ammonia titre not higher than 10%, even though higher titres are not to be excluded. It is also possible to add gaseous ammonia provided that the stirring of the liquid phase is such as to hinder the formation of too high local excesses especially in the organic phase. It is also possible to use instead of ammonia other alkaline substances such as, for example, primary, secondary or tertiary alkyl amines, provided that they are easily extractable from the organic phase by water washing.

Examples of amines are mono, di, trisubstituted alkyl C₁-C₆ amines. The methylamine and the dimethylamine can be cited.

The oxidizing agent must be able to react with the oxidizable products present in the raw reaction product without however interacting in a substantial way with the methylmethacrylate and with the methacrylic acid. Moreover, the oxidation by-products should be not difficult to be eliminated and also not polluting the monomer.

On the basis of the above mentioned requirements the skilled in the art is able to choose the oxidizing products known in the art which are satisfactory on the mentioned conditions. The hydrogen peroxide is the preferred oxidizing agent both due to its high oxidizing power and for the absence of by-products from the oxidation not acceptable from the ecologic point of view or of the process as indicated hereinabove. It is suitable to use diluted hydrogen peroxide, or diluted oxidizing agents in general, to avoid great local excesses. However it is also possible to use concentrated solutions of oxidizers, provided that one acts in such a way as to have a quick homogenizing of the treated organic phase.

The hydrogen peroxide, or the oxidizing agent in general, is mixed to the raw methylmethacrylate before contacting ammonia, or amine, preferably employed in the diluted form. The preferred amount of oxidizer varies according to the composition of the raw monomer. Generally the utilized amount is comprised between 10 and 2000 ppm.

The amount of H₂O₂, or of the oxidizing agent in general, is such as not to exceed the amount equivalent to the reducing substances present in the raw monomer.

The Applicant has found that it is possible to calculate this amount by measuring the SO₂ present in the raw product.

The H₂O₂ to be added is such that the residual SO₂ does not exceed a value of 200 ppm and anyway that the H₂O₂ is never present as such in the system.

The skilled in the art is able by following such indications to easily determine the suitable amount of the oxidizer to be employed.

By acting in this way, possible and casual polimerizations during extraction are avoided.

The inhibiting system comprises an inhibitor, or a mixture of inhibitors, to be introduced in the organic phase in amounts comprised between 1 and 200 ppm. The utilizable inhibitors belong to the most common classes of substances having such activity, for example phenols, phenolic ethers, quinones, aromatic amines, heterocyclic aromatic substances used alone or in mixture. Examples of inhibitors are: hydroquinone, hydroquinone monomethylether, pyrogallol, resorcin, p-t.butyl-catecol, p-phenilendiamine, diphenyl-p-phenylendiamine, anthracene, phenothiazine, methylene blue, etc. The preferred system is a mixture of phenothiazine and hydroquinone in a ratio comprised between 5:1 and 1:5 by weight. The amount of washing water can vary within wide limits.

The washing of the raw methylmethacrylate according to the present invention, can be carried out in different ways. one can for example use a liquid-liquid counter-stream extraction column in which there are feeded the light phase already oxidized and the ammonia from the bottom, and the washing water for the excess of alkali and methanol from the head; or one can operate in semicounter-current in a series of liquid-liquid separators-decanters, commonly known as Fiorentine separators in which the oxidized organic phase and the ammonia are feeded at the first stage and the washing water in the last stage.
Generally the stages are at least three, preferably from 5 to 8.

The invention will be illustrated by some examples which are not to be intended as limiting the present inverttion.

### EXAMPLE 1 (comparative)

7 g of an ammonia aqueous solution at 10% are added to 330 g of raw monomer deriving from the esterification of the methacrylamide sulphate in a plant of production of methylmethacrylate monomer and having the following average composition:

| | |
|---|---|
| Methylmethacrylate | 60-70% |
| Methanol | 18-24% |
| Water | 8-10% |
| Methacrylic acid | 0.5-1% |
| Other organic compounds | 2-4% among which acetone, dimethylether |
| Sulphurous anhydride | 1000 ppm |
| Hydroquinone (inhibitor) | 30 ppm, |
| thus obtaining a pH of about 9. | |

Such a mixture is sequentially extracted/washed four times with 125, 60, 60, 60 g, respectively, of aqueous solutions having the following average compositions:

| | Solut. 1 | Solut. 2 | Solut. 3 | Solut. 4 |
|---|---|---|---|---|
| Water | 70-80% | 80-90% | 90-95% | 100% |
| Methanol | 15-25% | 5-15% | 3-5% | |
| Methylmethacrylate | 3-6% | 1-4% | 1-3% | |
| Other organic materials | 2-4% | 1-2% | 1-2% | |

The first three aqueous solutions are drawn, respectively, from the 2^{nd}, 3^{rd} and 4^{th} stage, of the separator-decanter of the washing section with water in counter-current of the raw monomer deriving from the esterification of methacrylamide sulphate of a plant of production of methylmethacrylate.

The sequential extractions cannot occur as the separation between organic and aqueous layers is not sharp due to the presence of emulsions caused by substances formed during the extraction and having surface-active/emulsifying characteristics.
Besides this difficulty, also a partial polymerization of the contained monomers is obtained.

### EXAMPLE 2

Example 1 is repeated pre-treating the raw monomer with 450 mg of hydrogen peroxide at 33%, thus partially eliminating the reducing substances and till to a content of residual sulphurous anhydride after treatment of 150 ppm.
All the sequential extractions can be performed without problems as to the separation of the phases and without having polymerizations in the system.

### EXAMPLE 3 (comparative)

200 Kg/h of a 10% aqueous ammonia solution are added to about 11.000 kg/h of a solution coming from the esterification section of a plant of production of the methylmethacrylate monomer and having the average composition reported in example 1.
The resulting mixture, having a pH of about 8.5-9, is washed with about 3500 kg/h of water in counter-current in a series of 4 Fiorentine liquid-liquid separators-decanters.

After a period of 3 hours, the washing is to be interrupted owing to the presence of polymers in the various separators and owing to the impossibility to get the separation betwen the organic and aqueous layers.

### Example 4 (comparative)

Example 3 is repeated by adding also 30 ppm of phenothiazine in the raw monomer before introducing the ammoniacal solution. In such a way it is possible to have a good extraction for a period of time of 50 hours; the washing, however, also in this case is to be interrupted owing to the formation of polymers and to the difficulty to separate the phases in the separators.

### EXAMPLE 5

Example 4 is repeated, by gradually adding about 100 kg/h of an aqueous solution of a 2% hydrogen peroxide; so the residual contents of SO₂ in the raw monomer is brought under 200 ppm before adding ammonia.

By operating in this way it is possible to have an efficient and continuous extraction of the methacrylic acid and of the methanol for a period of 6 months, by definitively eliminating all the problems of polymerization and of emulsifiability of the phases.

## Claims

1. Process for the purification of raw methylmethacrylate obtained by reaction of acetoncyanhydrin reaction with an excess of concentrated sulphuric acid to form methacrylamide sulphate, then a transposition reaction at high temperatures is carried out for obtaining methacrylamide sulphate; then one carries out the esterification of the methacrylamide in sulphuric solution by dilution with water and by using methanol in excess; washing of the raw methylmethacrylate and subsequent distillation, characterized in that the washing is carried out with diluted ammonia or amines and at least an oxidizer capable to react with the oxidizable products of the reaction blank but not to react substantially with the methylmethacrylate and with methacrylic acid and operating in presence of inhibitors.

2. Process for the purification of raw methylmethacrylate according to claim 1, in which the washing with ammonia is carried out at room temperature and the ammonia is used in an amount higher than the stoichiometric one, with respect to the methacrylic acid present in the raw reaction mixture after the esterification phase.

3. Process for the purification of raw methylmethacrylate according to claims 1 and 2, in which the amount of ammonia is such as to maintain the pH between 7.5 and 9.5.

4. Process for the purification of raw methylmethacrylate according to the preceeding claims in which the ammonia is added as an aqueous solution.

5. Process for the purification of raw methylmethacrylate according to claim 1, in which primary, secondary or tertiary amines are employed which are easily extractable from the organic phase by water washing.

6. Process for the purification of raw methacrylate according to claim 1, by which the oxiding agent is chosen among the ones which are easily to be eliminated and which do not pollute the monomer.

7. Process for the purification of raw methacrylate according to claims from 1 to 6, in which the reaction raw mixture is treated with the oxidizing agent before adding ammonia or amine.

8. Process for the purification of raw methacrylate according to the preceeding claims, in which the amount of the oxidizing agent is such as not to exceed the amount equivalent to the reducing substances present in the distillate.

9. Process for the purification of raw methacrylate according to claim 8, in which the amount of the oxidizing agent is such as to maintain a concentration of residual SO₂ after washing between 1 and 200 ppm.

10. Process for the purification of raw methacrylate according to claims from 1 to 9, in which the oxidizing agent is hydrogen peroxide.

11. Proccess for the purification of raw methacrylate according to claims from 1 to 10, in which the inhibitor amount is comprised between 10 and 200 ppm.

12. Process for the purification of raw methacrylate according to claim 11, in which the inhibitor is a mixture of phenothiazine/hydroquinone in a ratio comprised between 5:1 and 1:5 by weight.

## Patentansprüche

1. Verfahren zur Reinigung von rohem Methylmethacrylat, erhalten durch Umsetzung von Acetoncyanhydrin mit einem Überschuß an konzentrierter Schwefelsäure zu Methacrylamidsulfat, anschließend Ausführung einer Transpositionsreaktion bei hohen Temperaturen zur Gewinnung von Methacrylamidsulfat; dann Durchführung der Veresterung des Methacrylamids in Schwefelsäurelösung durch Verdünnen mit Wasser und unter Verwendung von Methanol im Überschuß; Waschen des rohen Methylmethacrylats und anschließende Destillation, dadurch gekennzeichnet, daß das Waschen mit verdünntem Ammoniak oder Aminen und mindestens einem Oxidationsmittel, das in der Lage ist nur mit den oxidierbaren Produkten des Reaktionsansatzes zu reagieren, jedoch im wesentlichen nicht mit Methylmethacrylat und mit Methacrylsäure, ausgeführt wird und in Gegenwart von Inhibitoren gearbeitet wird.

2. Verfahren zur Reinigung von rohem Methylmethacrylat nach Anspruch 1, wobei das Waschen mit Ammoniak bei Raumtemperatur ausgeführt wird und das Ammoniak in einer Menge verwendet wird, die höher als die stöchiometrische, bezogen auf die vorliegende Methacrylsäure in dem rohen Reaktionsgemisch nach der Veresterungsphase, ist.

3. Verfahren zur Reinigung von rohem Methylmethacrylat nach Ansprüchen 1 und 2, wobei die Menge an Ammoniak derart ausgelegt ist, daß der pH-Wert zwischen 7,5 und 9,5 gehalten wird.

4. Verfahren zur Reinigung von rohem Methylmethacrylat nach einem der vorangehenden Ansprüche, wobei das Ammoniak als eine wässerige Lösung zugegeben wird.

5. Verfahren zur Reinigung von rohem Methylmethacrylat nach Anspruch 1, wobei primäre, sekundäre oder tertiäre Amine angewendet werden, die leicht aus der organischen Phase durch Waschen mit Wasser extrahierbar sind.

6. Verfahren zur Reinigung von rohem Methacrylat nach Anspruch 1, wobei das Oxidationsmittel ausgewählt ist unter jenen, die leicht zu beseitigen sind und die das Monomer nicht verunreinigen.

7. Verfahren zur Reinigung von rohem Methacrylat nach Ansprüchen 1 bis 6, wobei das rohe Reaktionsgemisch mit dem Oxidationsmittel vor der Zugabe von Ammoniak oder Amin behandelt wird.

8. Verfahren zur Reinigung von rohem Methacrylat nach den vorangehenden Ansprüchen, wobei die Menge an Oxidationsmittel derart ausgelegt ist, daß sie die Äquivalentmenge der in dem Destillat vorliegenden zu reduzierenden Substanzen nicht übersteigt.

9. Verfahren zur Reinigung von rohem Methacrylat nach Anspruch 8, wobei die Menge des Oxidationsmittels derart ausgelegt ist, daß eine Konzentration von restlichem SO₂ nach dem Waschen zwischen 1 und 200 ppm beibehalten wird.

10. Verfahren zur Reinigung von rohem Methacrylat nach Ansprüchen 1 bis 9, wobei das Oxidationsmittel Wasserstoffperoxid ist.

11. Verfahren zur Reinigung von rohem Methacrylat nach Ansprüchen 1 bis 10, wobei die Inhibitormenge zwischen 10 und 200 ppm umfaßt.

12. Verfahren zur Reinigung von rohem Methacrylat nach Anspruch 11, wobei der Inhibitor ein Gemisch von Phenothiazin/Hydrochinon in einem Gewichtsverhältnis, umfassend zwischen 5:1 und 1:5, ist.

## Revendications

1. Procédé pour la purification de méthacrylate de méthyle brut obtenu par réaction d'acétonecyanhydrine avec un excès d'acide sulfurique concentré pour former du méthacrylamide sulfate, puis une réaction de transposition à des températures élevées est effectuée pour obtenir le méthacrylamide sulfate ; ensuite on conduit l'estérification du méthacrylamide en solution sulfurique par dilution avec de l'eau et en utilisant du méthanol en excès ; lavage du méthacrylate de méthyle brut et distillation ultérieure, caractérisé par le fait que le lavage est conduit avec de l'ammoniac dilué, ou des amines, et au moins un agent oxydant capable de réagir avec les produits oxydables du brut de réaction, mais non de réagir de façon substantielle avec le méthacrylate de méthyle et avec l'acide méthacrylique et en opérant en présence d'inhibiteurs.

2. Procédé pour la purification de méthacrylate de méthyle brut, selon la revendication 1, dans lequel le lavage avec l'ammoniac est effectué à la température ambiante, et l'ammoniac est utilisé dans une quantité supérieure à la quantité stoechiométrique, par rapport à l'acide méthacrylique présent dans le mélange réactionnel brut après la phase d'estérification.

3. Procédé pour la purification de méthacrylate de méthyle brut, selon l'une des revendications 1 et 2, dans lequel la quantité d'ammoniac est telle qu'elle maintienne le pH entre 7,5 et 9,5.

4. Procédé pour la purification de méthacrylate de méthyle brut, selon l'une des revendications précédentes, dans lequel l'ammoniac est ajouté sous la forme d'une solution aqueuse.

5. Procédé pour la purification de méthacrylate de méthyle brut, selon la revendication 1, dans lequel des amines primaires, secondaires ou tertiaires sont employées, lesquelles peuvent facilement être extraites de la phase organique par lavage avec de l'eau.

6. Procédé pour la purification de méthacrylate de méthyle brut, selon la revendication 1, suivant lequel l'agent oxydant est choisi parmi ceux qui sont faciles à éliminer et qui ne polluent pas le monomère.

7. Procédé pour la purification de méthacrylate de méthyle brut, selon l'une des revendications 1 à 6, dans lequel le mélange brut réactionnel est traité par l'agent oxydant avant l'addition d'ammoniac ou d'amine.

8. Procédé pour la purification de méthacrylate de méthyle brut, selon l'une des revendications précédentes, dans lequel la quantité de l'agent oxydant est telle qu'elle ne dépasse pas la quantité équivalente aux substances réductrices présentes dans le distillat.

9. Procédé pour la purification de méthacrylate de méthyle brut, selon la revendication 8, dans lequel la quantité de l'agent oxydant est telle qu'elle maintienne une concentration de SO₂ résiduel après lavage entre 1 et 200 ppm.

10. Procédé pour la purification de méthacrylate de méthyle brut, selon l'une des revendications 1 à 9, dans lequel l'agent oxydant est le peroxyde d'hydrogène.

11. Procédé pour la purification de méthacrylate de méthyle brut, selon l'une des revendications 1 à 10, dans lequel la quantité d'inhibiteur est comprise entre 10 et 200 ppm.

12. Procédé pour la purification de méthacrylate de méthyle brut, selon la revendication 11, dans lequel l'inhibiteur est un mélange de phénothiazine/hydroquinone dans un rapport compris entre 5:1 et 1:5 en poids.
